# EUROPEAN PATENT APPLICATION

(11) **EP 2 719 400 A2**
(43) Date of publication of application: **16.04.2014**
(21) Application number: 12797636.3
(22) Date of filing: 08.06.2012
(51) Int. Cl.: A61L 27/10, A61L 27/54, A61L 27/58, A61F 2/28, C01B 37/00, A61P 19/00

(54) **BIOCERAMIC MATERIALS FOR TREATING OSTEOMYELITIS**

(30) Priority: 09.06.2011 ES 201100655 P
(71) Applicant: Universidad Complutense De Madrid, 28040 Madrid (ES)
(72) Inventor: VALLET REGI, María, E-28040 - Madrid (ES); MANZANO GARCÍA,Miguel, E-28040 - Madrid (ES); ESTEBAN MORENO, Jaime, E-28015 - Madrid (ES); MOLINA MANSO, Diana, E-28015 - Madrid (ES); GOMEZ BARRENA, Enrique, E-28015 - Madrid (ES)
(74) Representative: Carpintero Lopez, Francisco
(86) International application number: PCT/ES2012/000160
(87) International publication number: WO 2012/168512

(57) **Abstract**

The present invention describes the preparation of ordered mesoporous silica materials, capable of promoting bone development, and the use thereof as a release system for releasing a combination of antibiotics for treating bone infections locally and effectively.

The present invention therefore proposes the use of these materials for the treatment of osteomyelitis, since their high porosity and large specific surface area allows adsorbing a large amount of antibiotics for subsequently releasing them in a modulated manner, depending on patient's requirements. The present invention proposes the use of bioceramic SBA-15 for the adsorption of a combination of three antibiotics, the antimicrobial activity of which has been widely recognized: vancomycin, linezolid and rifampicin.

## Description

### Technical Field

The present invention is encompassed within the technical field of the manufacture of materials for bone implants with controlled-release of bioactive agents. More specifically, the invention relates to the manufacture of ordered mesoporous silica materials that promote bone development while at the same time release antibiotics for treating bone infection. Therefore, these new materials can be used in clinical cases of bone infection such that the local release of a combination of antibiotics allows mitigating the systemic dose of antibiotics conventionally used in the treatment of these diseases.

### State of the Art

Osteomyelitis is a bone tissue infection caused by various microorganisms, fundamentally bacteria. Even though it is a long-known disease, bone infection remains a challenge for modern medicine due to the great problems it presents not only to patients with bone infection, but to the entire health system. Osteomyelitis is a destructive inflammatory infection that results in the appearance of an encapsulated cavity called a sequestrum containing therein samples of devitalized bone tissue and a purulent content formed by inflammatory cells and microorganisms.

There are many types of osteomyelitis depending on the duration, mechanism of infection, portion of the affected bone and other factors, but in all cases the microbes most commonly responsible for osteomyelitis are: *Staphylococcus aureus, Streptococcus pyogenes, Mycobacterium tuberculosis* and Gram negative bacteria (particularly *Enterobacteriaceae,* including *Salmonella sp.* and *Pseudomonas aeruginosa).* Antimicrobial agents commonly used to fight said microbes are fluoroquinolones, clindamycin, rifampicin and cotrimoxazole because they penetrate the bone better. Beta-lactam antibiotics, glucopeptides and aminoglycosides diffuse < 20% so their doses must be high. The combination of several antibiotics is also usually effective to prevent resistance and to treat mixed infections.

The conventional treatment of this clinical picture includes a dual medical-surgical approach with the administration of antibiotics for long-term treatment and extirpation of devitalized bone tissue using a filler material stimulating the growth of new bone tissue. Extirpation of necrotic tissue is an essential procedure, but the intravenous and/or oral administration of antibiotics for 4 to 6 weeks has the drawback of spreading the antibiotics throughout the patient's body. This, along with the need of high doses due to the difficulty of the antibiotic in reaching the bone, means that there are more likely potential side effects. All this does not only entail discomfort for the patient but rather a high economic cost.

Therefore, considering alternative strategies for the treatment of osteomyelitis remains a challenge today. Local and controlled drug release is a new therapeutic modality that has been studied in recent years as it allows dosing high concentrations of antibiotics at the point of infection, preventing systemic toxicity (S.K. Nandi et al. "Local Antibiotic delivery systems for the treatment of osteomyelitis - A review", Materials Science and Engineering C 29 (2009) 2478-2485). Both non-biodegradable and biodegradable materials have been used as antibiotic carriers.

Among non-biodegradable material, the most commonly used is polymethylmethacrylate, PMMA, (H. van de Belt et al. "Infection of orthopaedic implants and use of antibiotic-loaded bone cements" Acta Orthop Scand 72 (2001) 557-571) in which antibiotics, such as gentamicin (K. Klemm "The use of antibiotic-containing bead chains in the treatment of chronic bone infections", Clinical Microbiology and Infectious Diseases 7 (2001) 28-31) or vancomycin (K. Shinsako et al. "Effects of bead size and polymerization in PMMA bone cement on vancomycin release" Bio-Medical Materials and Engineering 18 (2008) 377-385), have been introduced. Although the PMMA-antibiotic combination is effective for the treatment of osteomyelitis, its non-biodegradability means that these systems must be removed in a second surgical procedure, with the drawbacks that this entails both for the patient and for the health system.

In contrast, in addition to allowing high concentrations of antibiotic agents at the site of implant, biodegradable materials do not require a second intervention in order to remove same. Additionally, and as an added value, the biodegradable implant can be used as a filler material occupying the area left by the necrotic bone tissue, and gives rise to the formation of new bone tissue and guides the repair as it gradually degrades. To achieve this effect, the material used for the implant must have regenerative capacity. This approach is the trend in current clinical investigation, in what has become known as regenerative medicine. Among biodegradable materials, various materials have been used, such as collagen sponges impregnated with gentamicin (T. Ipsen "Gentamicin-collagen sponge for local applications" Acta Orthop Scand 62(6) (1991) 592-594), polymers derived from lactic acid with fluorinated quinolones such as pefloxacin (K. Kaellakopoulou "Treatment of experimental osteomyelitis caused by methicillin-resistant Staphylococcus aureus with a biodegradable lactic acid polymer releasing pefloxacin" Journal of Antimicrobial Chemotherapy 46 (200) 311-314), bioceramics such as hydroxyapatite cements, brushite cements or apatite xerogel where vancomycin is introduced (P.J. Ping et al. "Comparing the efficacy of three bioceramic matrices for the release of Vancomycin hydrochloride" Journal of Biomedical Materials Research Part B-Applied Biomaterials 93B(1) (2010) 51-58) or bioglass also with vancomycin (US 7,223,414). Document EP1671661 describes a bone substitute material composed of a mixture formed by granules of calcium sulfate dihydrate and calcium carbonate which contains one or several antibiotics effective against osteomyelitis that are sustained-released for a period of several days up to two weeks.

Many of the materials used as carriers for the controlled-release of drugs can be used as bone implants because they are biodegradable and biocompatible but some materials do not have enough mechanical strength and only have osteoconductive properties (act as a structural support in the formation and growth of new bone) but not osteoinductive (do not promote the binding of specific cells on bone tissue) or osteogenic properties.

Therefore, in order to prevent and/or treat a possible bone infection it would be desirable to obtain a bone filler material that is biodegradable, biocompatible, with osteoconductive, osteoinductive and osteogenic properties while at the same time allows the controlled-release of antimicrobial agents.

Ordered mesoporous silica-based materials have generated great interest as drug release systems since they were first used for this purpose in 2001 (Vallet et al. "A new property of MCM-41. Drug delivery system" Chemistry of materials 13(2) (2001), 308-311). The reasons for such interest are their structural and chemical properties: (1) high pore volume which allows confining a large amount of drug or bioactive agent; (2) large surface area which entails a high drug adsorption potential; (3) ordered pore distribution favoring homogeneity and reproducibility of drug adsorption and subsequent release: and (4) high density of surface silanol groups allowing the possibility of chemically modifying the pores of the wall in a simple manner, allowing a greater drug release control (Vallet-Regí et al. "Mesoporous Materials for Drug Delivery" Angewandte Chemie, Int. Ed. 46 (2007) 7548-7558), (Manzano et al., "Drug delivery from ordered mesoporous matrices" Expert Opin. Drug Deliv. 6(12) (2009) 1383-1400), (Manzano and Vallet-Regí, "New developments in ordered mesoporous materials for drug delivery" J. Mater. Chem., 20 (2010) 5593-5604). Furthermore, these materials can act as cell matrices where proteins, peptides or growth factors that can be subsequently released promoting cell proliferation and differentiation, can be easily incorporated (Vallet-Regí et al., "Osteostatin-loaded bioceramics stimulate osteoblastic growth and differentiation", Acta Biomaterialia 6 (2010) 797-803), (Vallet-Regí et al., "The osteoinductive properties of mesoporous silicate coated with osteostatin in a rabbit femur cavity defect model" Biomaterials 31 (2010) 8564-8573). The combination of these (structural, chemical and bioactive) characteristics turns the materials into materials of great biomedical interest as demonstrated by more than a hundred publications.

Today, work continues to be conducted in three directions for the purpose of obtaining mesoporous materials capable of releasing drugs while at the same time regenerating bone tissue: (1) surface functionalization through organic groups to perform drug adsorption and subsequent drug release in a controlled manner, (2) macroporosity to allow cells to bind inside the scaffolds and (3) chemical surface modification to enable covalent bonds of peptides and proteins that favor the bone regeneration on the outer surface of the scaffold (Manzano et al. "New developments in ordered mesoporous materials for drug delivery" J. Mater. Chem., 20 (2010) 5593-5604).

The present invention relates to bioceramics capable of treating a bone infection by means of the local release of antibacterial agents while at the same time regenerating bone tissue once they are implanted.

### Description of the Invention

### Brief Description of the Invention

The present invention proposes the use of ordered mesoporous silica materials which, due to their high porosity and large specific surface area, can adsorb and subsequently release antibiotics for the treatment of osteomyelitis in a modulated manner using specific functional groups anchored to the wall of the mesoporous silica by means of a simple chemical method. Therefore, quicker release or release for longer time periods can be obtained locally in the affected area without affecting healthy areas of the patient's body. With the mesoporous matrices it may even be possible to design a carrier capable of responding to external stimuli, such that drug release takes place when desired, in what has become known as Smart Drug Delivery Systems. Based on the data obtained until now, as a result of using these materials the release of high antibiotic concentrations at a short time and always above the "breakpoint" can be achieved, which means that the system will be working starting from the first moment of implantation to eliminate the infection quickly and effectively. Additionally, as demonstrated by this invention, any combination of antibiotics can be included inside the pores of the material, which will allow the customized development of release vectors, where the type of antibiotics to work with can be individually selected depending on the patient and/or type of infection. This technology is in the line with the technology used by large pharmaceutical companies today, where individualized therapies and treatments are increasingly being adjusted to the patient's characteristics. As an added value and in combination with the antibiotics, a bacterial quorum sensing inhibiting peptide called a RIP (RNA Inhibiting Peptide) can be added inside the pores, said peptide being capable of interfering in the creation of biofilm which the bacteria produce around themselves for protection, and therefore making the antibacterial treatments more effective.

A filler material which, in addition to fighting against infection in a quick and effective manner, will stimulate the growth of new bone tissue that can be modulated depending on the functional groups covering bioceramic, is therefore obtained. This technology will allow removing the necrotic area and implanting a material that favors bone regeneration, so it would fall under the technology which has become known as Regenerative Biomedicine, and which is acquiring a great deal of interest in recent years within biomedicine.

### Detailed Description of the Invention

The present invention relates to the use of certain bioceramics for the treatment of bone tissue infection. Specifically, it proposes the use of ordered mesoporous silica-based materials, which have been the focus of several researches for use as drug controlled-release systems due to their superb physicochemical properties. Specifically, their textural properties such as the high pore volume, large specific surface area and the ordered distribution of that pore network, have encouraged the use thereof in designing drug release systems. Additionally, the high concentration of silanol groups on the surface of these materials allows the quick and easy anchoring of different functional groups, as has been extensively described in the literature (M. Froba et al., Angew. Chem. Int. Ed., 2006, 45, 3216-3251), in order to have better control over drug release and bone regeneration kinetics.

Therefore, the present invention proposes the use of these materials for the treatment of osteomyelitis, since their high porosity and large specific surface area allows adsorbing a large amount of antibiotics for subsequently releasing them in a modulated manner, depending on patient's requirements. The present invention proposes the use of bioceramic SBA 15 for the adsorption of a combination of three antibiotics the antimicrobial activity of which has been widely recognized: vancomycin, linezolid and rifampicin.

Therefore, when a patient with bone infection is to be treated, and after the surgeon removes the devitalized tissue, implanting the aforementioned bioceramic loaded with the combination of the three antibiotics is proposed, such that these pharmaceutical agents will be released on the exact site of infection, preventing the side effects of a long-term systemic administration. Therefore, high concentrations of these antibiotics will be obtained in the site of infection, so the bioceramics will be fighting against the infection in a quick and effective manner from the start of the implantation.

Additionally, and as an added value of such bioceramics, the growth of new bone tissue will be stimulated, the space produced by the elimination of infected bone being filled instantly, for the subsequent regeneration of the bone tissue in a progressive manner, as has been recently demonstrated by the inventors by coating mesoporous silica with osteostatin (Trejo et al., Biomaterials, 2010, 31, 8564-8573). Therefore, newly formed bone tissue with complete absence of infection is anticipated at the end of the treatment.

### Brief Description of the Drawings

Figure 1 describes the release of antibiotics (linezolid, rifampicin and vancomycin) from a porous bioceramic SBA-15 over time.
Figure 2 depicts the biological activity of the three antibiotics released from porous bioceramic SBA-15 measured by means of biological method: (a) rifampicin, (b) vancomycin and (c) linezolid.

### Embodiment of the Invention

The invention is illustrated by means of the following example which does not limit the scope thereof.

### Example

### Preparing the ordered mesoporous material

By way of representative example, porous bioceramic SBA 15 having an ordered hexagonal pore distribution with sizes between 6 and 8 nm, and specific surfaces areas between 600 and 800 cm²/g, can be prepared. The synthesis of such bioceramics follows the method described by Zhao et al. (J. Am. Chem. Soc., 1998, 120, 6024-6036) and is based on the use of a surfactant that will act as a template and on which polycondensation of the silica source will occur. Specifically, the surfactant Pluronic P123 (4 g) is dissolved in 138 mL of water and 10.3 mL of hydrochloric acid. It is left uncovered at a temperature of 35°C and with constant stirring until the surfactant is completely dissolved. Next, 8.2 mL of tetraethyl orthosilicate (TEOS) are added, thus obtaining a molar composition of 1.0 TEOS: 0.017 P123: 208 H₂O: 3.4 HCl. After a few minutes, it is covered and left to react for 24 hours at 35°C in a sealed Teflon recipient. After this time it is introduced in an oven at 100°C for another 24 hours. The resulting material is filtered and left to dry for 24 hours at 60°C. The surfactant is eliminated by means of an extensively described extraction method, in which 100 mL of an absolute ethanol solution acidified with HCl is prepared for each gram of sample to be extracted. The solution is contacted with the material and is kept under reflux at 80°C for 5 hours. After that time, it is filtered and the process is repeated five more times to eliminate the largest possible amount of surfactant.

### Loading the antibiotics in the mesoporous material

Once the material is dried, it is loaded with three different antibiotics: linezolid, rifampicin and vancomycin. To that end, the antibiotics are loaded in SBA 15 tablets of 150 mg by means of introducing each of the tablets in a solution formed by 1/3 volume of the stock antibiotic solution and 2/3 volume of a sterile phosphate buffer solution (PBS, Sigma-Aldrich). The stock solutions of each of the antibiotics are at a concentration of 1 mg/mL. To combine two antibiotics, the loading solution is prepared with 1/3 volume of each of the antibiotics and 1/3 volume of PBS. To combine all the antibiotics, SBA 15 pieces of 150 mg are immersed in a solution formed by 1/3 volume of stock solution of each of the antibiotics. In any case, the pieces included in the loading solution are incubated at 4°C under stirring. After 24 hours, the pieces are transferred to a new plate and dried at 4°C. After this, the pieces are kept at 4°C until release.

### Releasing antibiotics in vitro

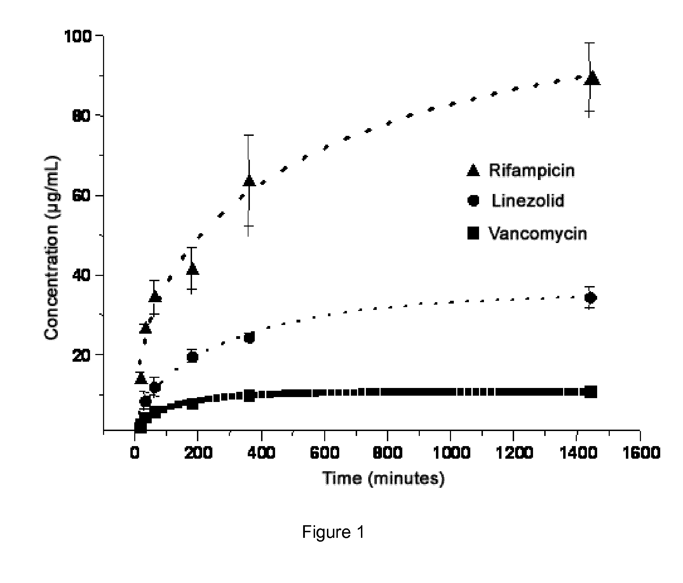

The release of the three antibiotics *in vitro* is shown in Figure 1, where a large amount of antibiotics is seen to be released in a short time period, which will cause a shock which presumably is very effective against bacterial infection. In fact, the concentration of antibiotics moments after the start of the release studies is always above the "breakpoint" for each of the antibiotics, which means that the drugs are effective and begin to eliminate the bacteria causing the infection from the start.

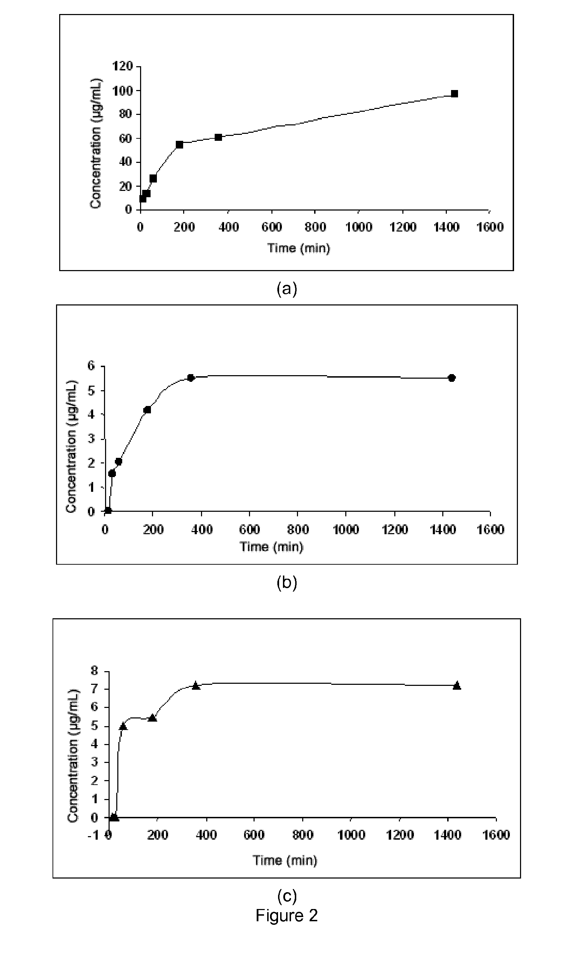

Likewise, by evaluating the activity of the antibiotics released by means of the biological method, it is obvious that the released antibiotics are effective in *vitro* (Figure 2).

## Claims

1. Antibiotic-containing bioceramic material with controlled-release of bioactive agents, **characterized in that** it comprises an ordered mesoporous silica material, one or several antibiotics suitable for the treatment of osteomyelitis and, optionally, a bacterial quorum sensing inhibiting peptide.

2. Antibiotic-containing bioceramic material with controlled-release of bioactive agents according to claim 1, where the ordered silica material is SBA-15.

3. Antibiotic-containing bioceramic material with controlled-release of bioactive agents according to the preceding claims, where the antibiotic is rifampicin, vancomycin, linezolid or a combination thereof.

4. Antibiotic-containing bioceramic material with controlled-release of bioactive agents, where the inhibiting peptide is RIP (RNA Inhibiting Peptide).

5. Use of the bioceramic material defined in claims 1-4 in the elaboration of a bone implant for the treatment of osteomyelitis.
